# EUROPEAN PATENT APPLICATION

(11) **EP 4 480 445 A2**
(43) Date of publication of application: **25.12.2024**
(21) Application number: 24210813.2
(22) Date of filing: 24.09.2020
(51) Int. Cl.: A61C 13/10

(54) **A METHOD FOR DIGITALLY DESIGNING A DENTURE USING AN EXISTING DENTURE**

(30) Priority: 27.09.2019 EP 19200306; 27.09.2019 US 201916585831
(62) Divisional of application: 20775017.5
(71) Applicant: 3Shape A/S, 1060 Copenhagen K (DK)
(72) Inventor: FISKER, Rune, 1060 Copenhagen K (DK)
(74) Representative: Guardian IP Consulting I/S

(57) **Abstract**

Disclosed are computer implemented methods and user interfaces for facilitating the design of a so-called copy denture, whereby an existing denture is reproduced after making changes to the existing denture to improve fit, aesthetics or other parameters.

## Description

### Technical Field

This disclosure generally relates to a computer implemented method and user interface for facilitating the design of a so-called copy denture, which typically involves a method whereby an existing denture is reproduced after making changes to the existing denture to improve fit, aesthetics or other parameters.

### Background

With the advancement of digital dentistry it is becoming more attractive to design and manufacture dentures using digital processes, such as scanning, designing and manufacturing.

One aspect of denture design and manufacturing is so-called copy dentures. This relates to a process where a patient already has a denture that generally works well, e.g. the bite and the aesthetics are good. However, for some reason the patient may need a new denture. Typically this can be due to bone retraction which is common for edentulous patients. Bone retraction results, leading to reduced fit between the gingiva and the denture, and therefore a new gingiva facing design of the denture is desired.

Providing a digital / computer implemented method of design and manufacture of dentures is of great interest, and in the following such a method will be disclosed and in particular a process that allows for improved copy dentures.

### Summary

In one aspect disclosed herein is a computer implemented method for generating a digital model of a denture comprising a digital model of a denture base and at least one digital model of denture teeth, wherein the method may comprise, in sequence, the steps of:
- obtaining a digital model of a fitted existing denture,
- segmenting the digital model of the fitted existing denture into an intermediate denture base model and at least one intermediate denture teeth model,
- modifying the anatomy of the intermediate denture base model,
- designing a coupling arrangement between the intermediate denture base model and the at least one intermediate denture teeth model, wherein the coupling arrangement comprises at least one recess formed in the intermediate denture base model and a protrusion extending from the at least one intermediate denture teeth model shaped to match the at least one recess, and
- generating a digital data file of a final denture base model based on the intermediate denture base model and generating a digital data file of at least one final denture teeth model based on the at least one intermediate denture teeth model.

The above method is particularly advantageous when the sequence of the steps above is maintained, especially when the anatomy of the intermediate denture base model is modified before the coupling arrangement is designed, as this allows for improved aesthetics while ensuring a secure coupling arrangement by preventing, or at least reducing, undercuts, which are generally unwanted.

In the current description the term "sequence" is used when a certain sequence of steps is desired in a specific order, although, in embodiments, additional steps may occur between the recited steps. For example, in the aspect above the sequence of steps needs to be maintained relative to each other, i.e.:
1. Obtain a digital model of the existing denture,
2. Segmenting the existing denture model into a denture base model and denture teeth model,
3. Modifying the anatomy of the denture base model,
4. Designing a coupling arrangement between the denture base model and the denture teeth model, and
5. Generating a digital data file of the denture base and the denture teeth.

In an alternative aspect, the steps need not be in sequence and can be switched around.

The term "existing" denture refers to an existing denture which the patient previously has or is using. The existing denture may be one where the patient is satisfied with the general performance of the denture, however some improvement is desired.

The existing denture is then fitted. By fitting the denture the denturist will typically perform an improvement on the existing denture. The denturist may test the improvement on the patient. For example, if the fit between the gum and the denture has deteriorated, e.g., due to bone retraction, the denturist may use the existing denture as a bite plate filling the gum facing surface with an alginate, silicone or similar impression material and ask the patient to bite down which generates an impression of the patients current gum profile on the existing denture.

Other fittings may, for example, be to repair the broken denture, e.g., a broken tooth. The fitting itself may simply be to verify the fit of the existing denture without making any physical modifications. Other modifications may be done digitally or during manufacturing, e.g., the user might like the fit of the existing denture but want more realistic teeth or gingiva colours which can be provide by alternative materials or production methods.

The fitted existing denture can then be scanned using different scanning techniques in order to provide a digital model of the fitted existing denture.

Another way of fitting may be in a case where the patient has lost the denture somehow but the digital data record of the existing (lost) denture is present or is recovered from the dentist. In this case the historical denture file would be used for manufacturing a temporary version (a so-called try-in) of the existing denture which can be used for the desired fitting and creating of the new digital dataset of the fitted existing denture model. Such a manufacturing could be 3D printing the historical template denture in a decent quality.

Furthermore, the term "fitted" is not always used in relation to the existing denture, however, it should be understood that when referring to the existing denture herein it is a reference to the fitted existing denture unless specifically stated otherwise.

The step of obtaining a digital model of an existing denture may thus comprise scanning the existing denture. This could be done by using a traditional dental laboratory scanner, where the both side of the existing denture is scanned and merged together to form at complete digital model. Another way could be to use an intro oral scanner and simply scanning the existing denture from 360° to obtain a complete model.

Alternatively the step of obtaining the digital model may simply comprise loading a digital data file of the digital model into the computer implemented method disclosed herein.

In the current disclosure the reference to a model implies a digital computer model present in the memory of a computer processing unit/device.

Accordingly, unless specifically stated that the model is a physical or manufactured model, a model as referred to herein is a digital model, typically stored as a data set forming a three dimensional model.

In the same context any action, modification or design done to such a model are digital in nature and performed in a digital design environment, for example through a user interface.

With the digital model of the fitted existing denture in the computer implemented method the digital model is segmented into an intermediate denture base model and at least one intermediate denture teeth model.

Segmentation can be done in different ways. In one embodiment the user identifies each tooth on the existing denture model, the computer implemented method may aid the user in this identification by applying shape recognition algorithms or machine learning.

Segmentation could also be done completely automatically, where each tooth is identified and segmented by means of sophisticated trained neural networks, machine learning, deep learning, etc.

The computer implemented method may subsequently identify a gum-tooth border spline which identifies the border between the denture tooth/teeth and the denture base of the existing denture model. The border spline can be determined manually by the user, a segmentation service using a neural network to determine the tooth and gingiva border can also be used to determine the border spline, edge detection on the meshes forming the existing denture model or other known means for determining edges and/or margin lines on a three dimensional digital model.

In another embodiment, the computer implemented method performs a process including slicing model into a plurality of 2D cross section elements perpendicular to the occlusion plane. These plurality of cross section slices are subjected to a curvature analysis in order to locate the tooth/gum boundary points for each slice. The points are mapped to the model and interconnected to form the boundary spline.

The gum-tooth border spline can subsequently be further modified manually if so desired.

A gum-tooth border spline may be generated for each tooth and can be used to segment that tooth from the denture base, so that each tooth forms a separate denture tooth model. I.e., the step of segmenting the denture base model comprises segmenting each tooth of the existing denture in the digital model of the fitted existing denture.

Alternatively, a gum-tooth border spline can alternatively be generated for a group of teeth whereby a group of teeth are segmented into a single denture teeth model. This may provide a number of denture teeth models each comprising a number of teeth. For example the anterior denture teeth may be grouped in one anterior denture teeth model, and the posterior teeth may be grouped in a first posterior denture teeth model and a second posterior denture teeth model for the side opposite the first posterior denture teeth model.

In yet an alternative embodiment one gum-tooth border spline is generated for all the teeth whereby one single denture teeth model is generated for the teeth.

The gum-tooth border spline(s) generated are then used to separate the existing denture model into an intermediate denture base model and one or more intermediate denture teeth models.

One advantage of segmenting into denture teeth models that comprises more than one tooth is that the final manufactured structure is stronger and more mechanically stable and also the relative orientation is maintained which facilitates and aids the placement of the final manufactured denture teeth in the final manufactured denture base.

However, in some applications the option of segmenting single teeth may provide more design freedom in the anatomical sculpting step as individual insertion direction may be used instead of obeying to a single insertion direction in the case of a one unit bridge segment including all the teeth.

Additionally the flexibility of designing segments for individual teeth may enable a more optimal material usage during manufacturing, if the teeth are for example milled from a block of material.

Accordingly, in one embodiment the step of segmenting the digital model comprises segmenting a group of at least two teeth of the fitted existing denture in the digital model of the fitted existing denture.

With the digital model of the existing denture segmented into an intermediate denture base model and at least one intermediate denture teeth model, the models can be individually modified.

Preferably the anatomy of the intermediate denture base model is modified. For example the papillas can be sculpted/modified. The papillas are the interdental gingiva between teeth.

Other gingiva anatomies may additionally or alternatively be modified, e.g. the mucogingival junction, the gingival groove etc. Alternatively or additionally, the intermediate denture teeth models can also be modified if necessary.

As discussed previously it is preferable to design the coupling mechanism between the denture base model and the denture teeth models after the anatomies of the models have been modified.

The coupling arrangement between the intermediate denture base model and the at least one intermediate denture teeth model are preferably designed as an at least one recess in the denture base model and a protrusion extending from each of the at least one intermediate denture teeth models, each protrusion is shaped to fit/match the corresponding recess formed in the denture base model.

In one embodiment, the step of designing a coupling arrangement may also comprise providing a cement gap between the at least one recess and the corresponding protrusion in the shape of an offset between a digital model of the at least one recess and a digital model of the at least one protrusion.

In one embodiment, the recess and protrusion are formed using the gum-tooth boundary spline as a reference for extruding a digital shape. The direction of the extrusion can be determined automatically, however, a user may determine the extrusion direction by identifying a so-called insertion direction of the denture teeth model. Accordingly, different insertion direction, and thereby extrusions direction, may be defined for those embodiments where there are several denture teeth models.

Such extrusion also provides the advantage that undercuts are prevented and in particular in aspects where the step of designing the coupling arrangement is performed after modifying the anatomy of the denture base model or denture teeth model any such modifications will not affect the ability to assemble the final physical denture base and the final physical denture teeth into a final physical denture.

Subsequent to the extrusions the at least one protrusion may further be modified if so desired. The user may specify additional couplings settings to shape the protrusion, such as the coupling depth, coupling angle, rounding radius and fillet radius in order to securely anchor the teeth in the physical denture base after manufacturing while taking into account design constrains such as material thickness of base etc. These settings could also be provided automatically from an optimization algorithm.

The recesses and protrusions may be designed simultaneously. Alternatively the protrusions may be designed and subsequently the recesses may be generated by subtracting the protrusion models from the denture base model. This can for example be done by a Boolean operation which is commonly known in the art. Additional adjustments can be applied to account for glue/cement space and drill compensation.

In another aspect, there is disclosed herein a method for manufacturing a denture comprising the computer implemented method disclosed herein, where the digital data file of the final denture base model and the digital data file of the at least one final denture teeth model are provided to a 3D manufacturing process such as printing or milling for manufacturing a denture base and at least one denture teeth and assembling the denture base and the at least one denture teeth, such as by gluing.

In a further aspect there is disclosed a user interface presenting a first user interface session on a digital monitor for grouping teeth models on a digital dental model wherein the first user interface session comprises a rendering of the digital dental model in a three dimensional workspace and a toolbar comprising at least one grouping button on the toolbar, wherein when activating the at least one grouping button at least one closed spline is generated enclosing at least one tooth at a boundary between at least one tooth model and a gingiva model of the digital dental model.

In one embodiment the user interface may comprise a plurality of grouping buttons arranged in the toolbar and when activated each of the grouping button will provide one or more of the following actions,
- generate a plurality of closed splines, each spline enclosing one tooth model of the digital dental model,
- generate one closed spline enclosing all teeth models of the digital dental model,
- generate a plurality of closed splines, each spline enclosing a group of tooth models of the digital dental model, and/or
- prompting the user to select one or more tooth models of the digital dental model to be enclosed by a spline of the at least one closed spline.

In one embodiment at least one tooth model is a denture teeth model and the digital dental model is a fitted existing denture model comprising the denture teeth model and a denture base model.

In a further embodiment a second user interface session comprises a rendering of the digital dental model in a three dimensional workspace wherein the digital dental model is segmented into a denture base model and the at least one tooth model based on the at least one closed spline.

For example, the digital dental model can be segmented into the denture base model and the at least one tooth model based on the at least one closed spline, by using the at least one closed spline as a cutting spline to cut the digital dental model into the denture base model and the at least one tooth model.

### Brief description of the drawings

The above and/or additional objects, features and advantages of the present disclosure, will be further described by the following illustrative and nonlimiting detailed description of embodiments of the present disclosure, with reference to the appended drawing(s), wherein:
Fig. 1 shows an example of a first user interface session of a scan session during a top side scan step of a method for building a digital design of a new denture;
Fig. 2 shows an example of a second user interface session of a scan session during a bottom side scan step of a method for building a digital design of a new denture;
Fig. 3 shows an example of a third user interface session involving merging during a method for building a digital design of a new denture;
Fig. 4 shows an example of a fourth user interface session during a teeth identification step of a method for building a digital design of a new denture;
Fig. 5 shows an example of a fifth user interface session during a segmentation step of a method for building a digital design of a new denture;
Fig. 6 shows an example of a sixth user interface session involving grouping during a method for building a digital design of a new denture;
Fig. 7 shows an example of a seventh user interface session during a first sculpting step of a method for building a digital design of a new denture;
Fig. 8 shows an example of an eighth user interface session during a second sculpting step of a method for building a digital design of a new denture;
Fig. 9 shows an example of a ninth user interface session during a coupling mechanism step of a method for building a digital design of a new denture;
Fig. 10 shows an example of a tenth user interface session during a coupling mechanism step of a method for building a digital design of a new denture;
Fig. 11 shows an example of an eleventh user interface session involving Boolean subtraction during a method for building a digital design of a new denture; and
Fig. 12 shows an example of a twelfth user interface session involving model finalization during a method for building a digital design of a new denture.

### Detailed description

In the following description, reference is made to the accompanying figures, which show by way of illustration how the disclosure may be practiced.

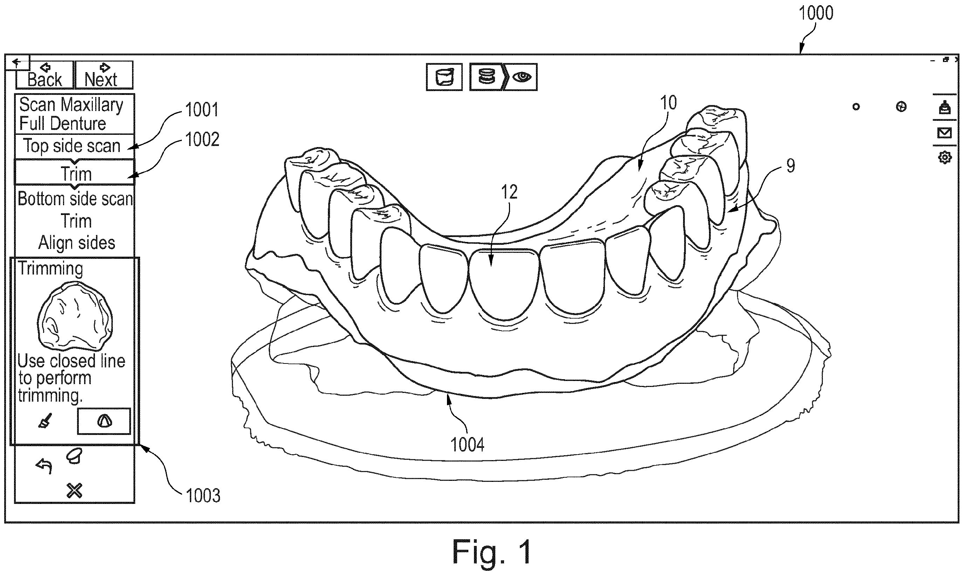

Figs. 1-12 show examples of a method as disclosed herein wherein a fitted existing denture is used to build a digital design of a new denture which is ready to be manufactured using 3D printing or milling.

Figs. 1-3 show how a digital model of a fitted existing denture can be obtained.

A first user interface session 1000 of a scan session is shown in Fig.1. The occlusal side 10 of an existing denture 9 including teeth 12 has been scanned during a top side scan step 1001.

The occlusal side scan 10 is being trimmed in a trim step 1002 using trim tool 1003, such as a closed line 1004 for trimming.

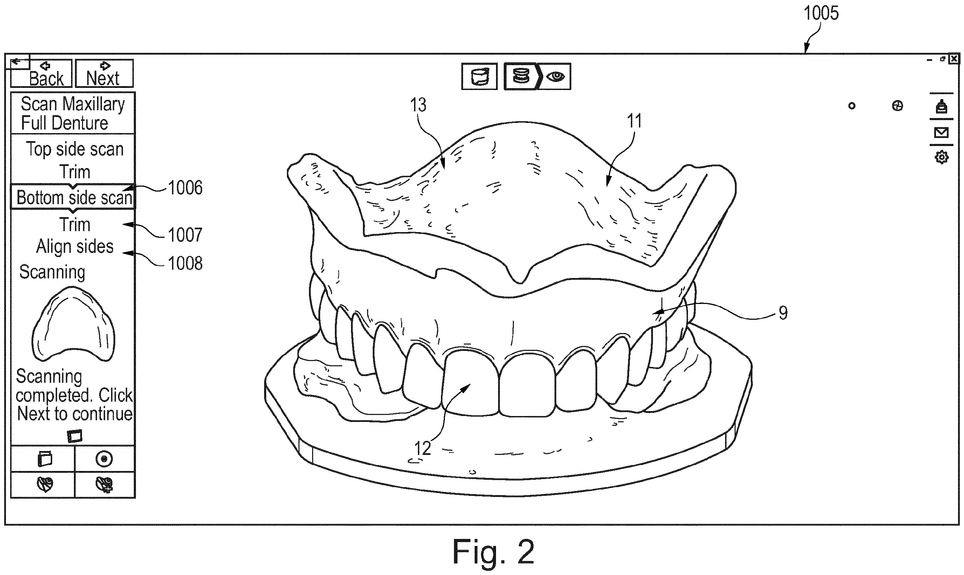

After trimming of the occlusal side scan a gingiva side scan 11 from a scan session is provided in a second user interface session 1005 in a bottom side scan step 1006 as shown in Fig. 2.

An impression material 13 has been used in the existing denture 9 and used to do an impression of the patient's current gum surface in order to provide a fitted existing denture, which is the denture 9 being scanned as disclosed with respect to Figs. 1 and 2.

Similar to the trim step 1002 of the occlusal side scan 10 a trim step 1007 is also performed where the gingiva side scan 11 is trimmed by a user.

Finally the occlusal side scan 10 and the gingiva side scan 11 are aligned in an alignment step 1008. In exemplary embodiments, this can be performed automatically seeing that the teeth 12 of the scans can be used as overlapping surfaces for alignment reference.

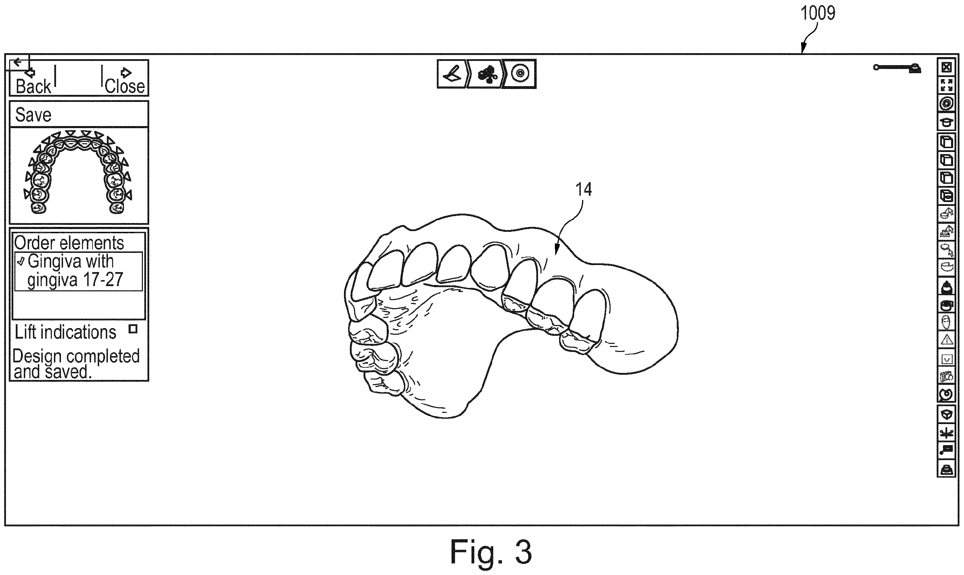

The aligned occlusal side scan 10 and gingiva side scan 11 are then merged into a digital model of the fitted existing denture 14 as shown in a third user interface session 1009 as shown in fig.3.

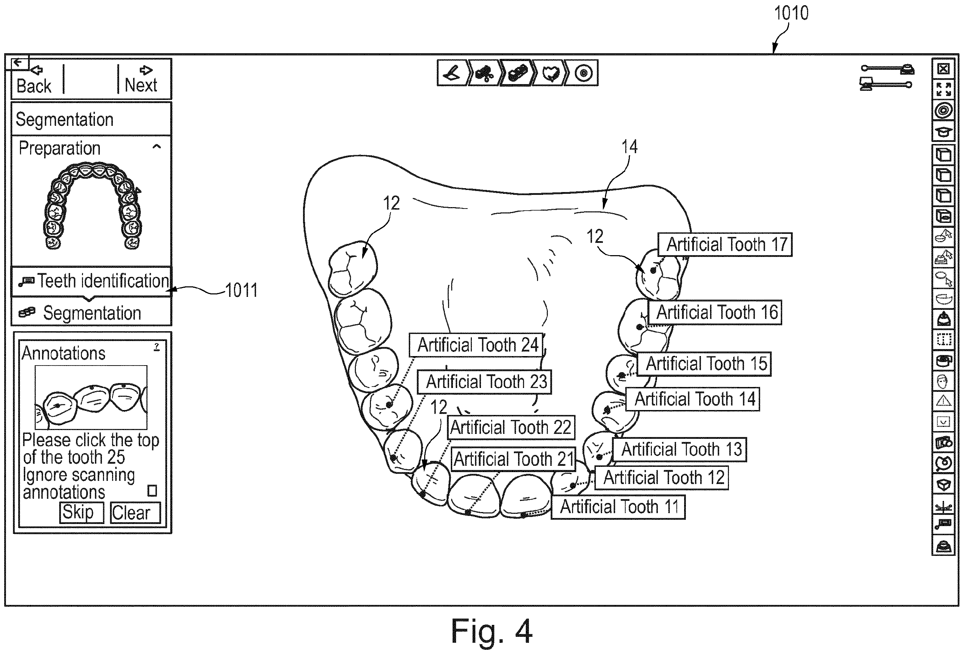

A segmentation process is subsequently initiated as shown in Figs. 4-6.

Fig. 4 shows a fourth user interface session 1010 whereby each separate denture/artificial tooth 12 is identified on the digital model of the fitted existing denture 14 in a teeth identification step 1011.

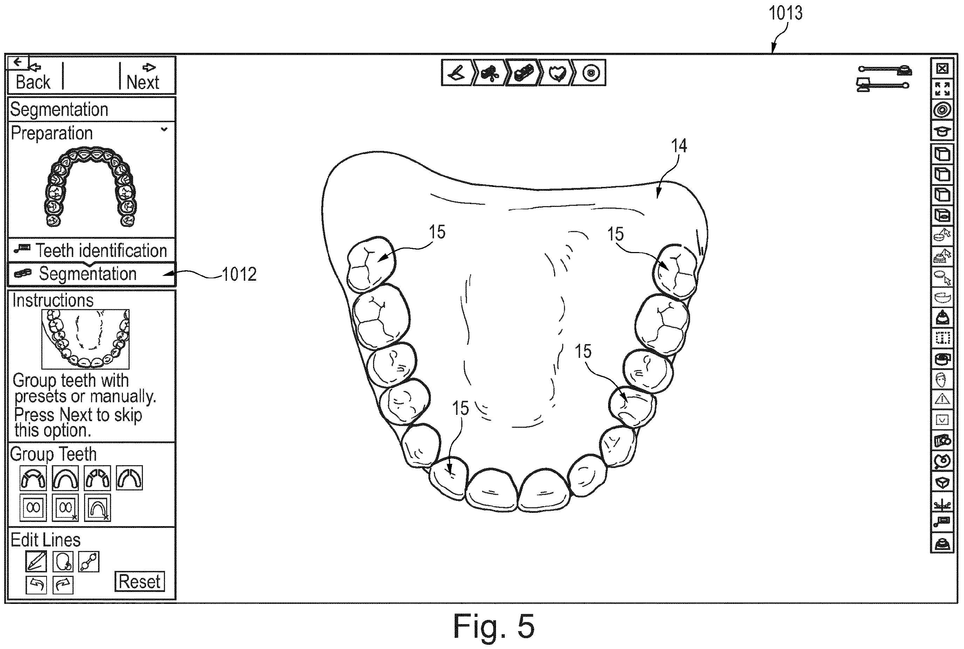

Subsequent to the teeth identification 1011 a gum-tooth spline 15 is derived for each tooth 12 in segmentation step 1012 in a fifth user interface session 1013 shown in Fig. 5.

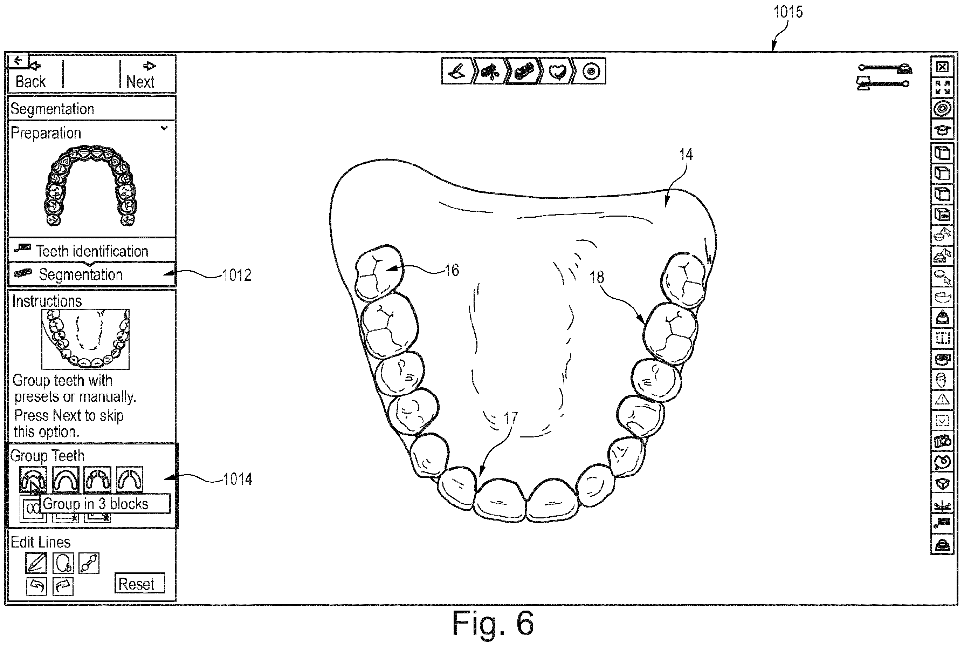

In the segmentation step 1012 the user can chose to group the teeth into groups by using grouping tools 1014 as shown in a sixth user interface session 1015 shown in Fig. 6. In the current case the user choses to generate three groups which results in a first, second and third gum-tooth splines 16, 17 and 18 being generated. Each gum-tooth is a closed spline that defines the tooth boundary of a group of teeth.

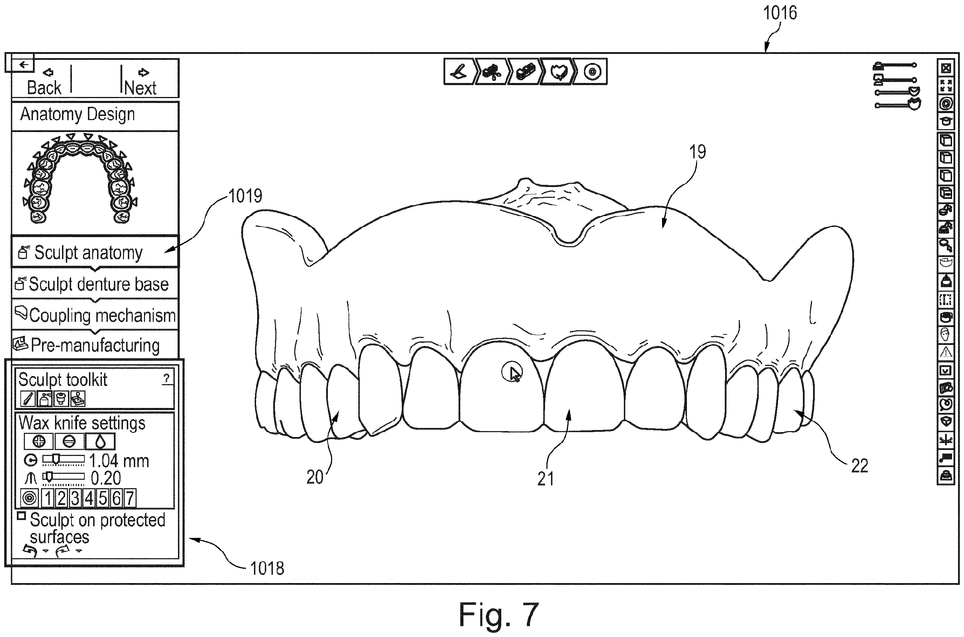

Using the gum-tooth splines 16, 17 and 18 the digital model of the fitted existing denture 14 can be segmented into an intermediate denture base model 19, a first intermediate teeth model 20, a second intermediate teeth model 21 and a third intermediate teeth model 22 as shown in a seventh user interface session 1016 shown in Fig. 7.

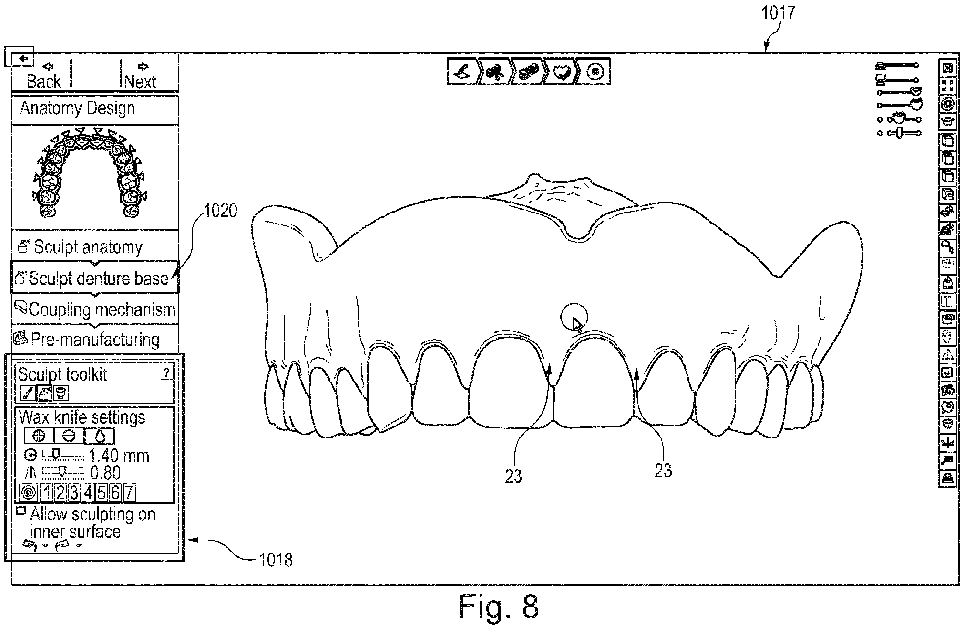

Figs. 7 and 8, showing the seventh user interface session 1016 and an eighth user interface 1017,which illustrates how the anatomies of the intermediate denture base model 19 and the intermediate teeth models 20, 21 and 22 can be modified in two sculpting steps 1019 and 1020. For example, the papillas 23 of the denture base in Fig. 8 have been accentuated by digitally modifying them using the sculpt toolkit 1018.

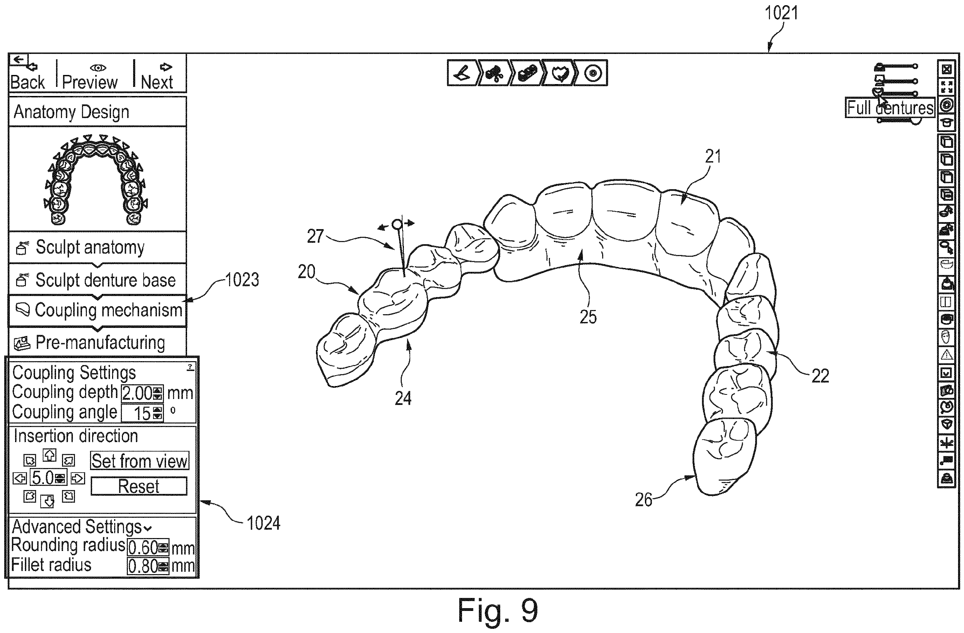

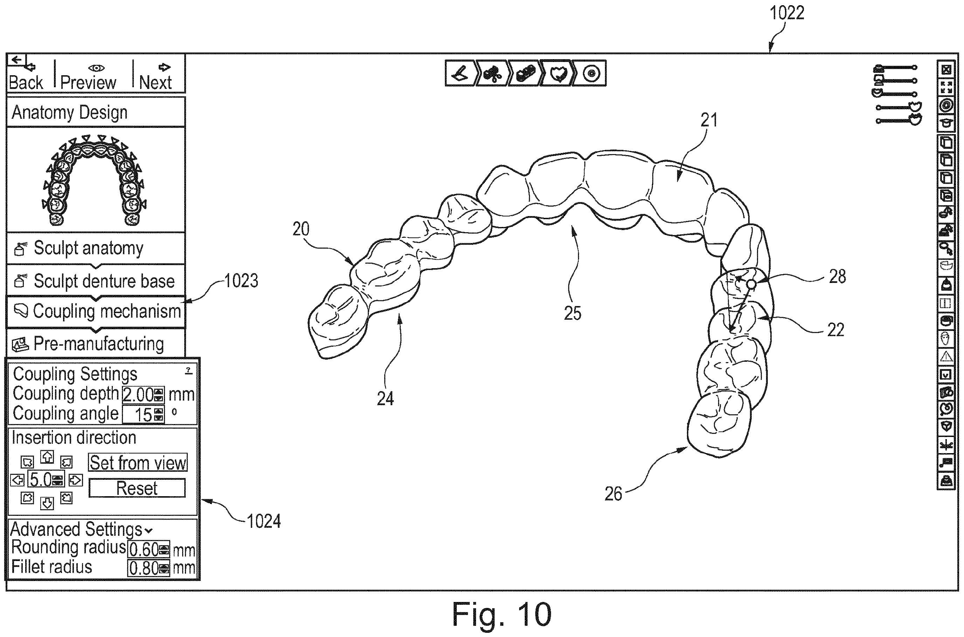

After the anatomies (outer visible surfaces) of the intermediate denture base model 19 and the intermediate denture teeth models 20, 21, 22 have been modified to provide a satisfactory result, a coupling mechanism is generated in a coupling mechanism step 1023 as shown in user interface session 1021 in Fig. 9 and user interface session 1022 in Fig. 10.

Figs. 9 and 10 show the intermediate denture teeth models 20, 21 and 22 shown independently from the denture base. Protrusions 24, 25 and 26 are generated based on the respective gum-tooth border splines 16, 17 and 18. The protrusions extend below the anatomy of the teeth and do not need to be aesthetically correct.

In Fig. 9 the insertion direction 27 of the first intermediate denture teeth model 20 is determined and the protrusions 24 have been reduced in design compared to the protrusions 25 and 26 of the second and third intermediate denture teeth models 21 and 22.

Similarly, as shown in Fig. 10, the protrusions 25 and 26 of the second and third intermediate denture teeth models 21, 22 are designed similarly to the first protrusion 24. Also, the insertion direction 28 of the third intermediate denture teeth model 22 is determined. Seeing that the denture teeth are provided in groups as discussed above, different insertion directions can be provided which can be an advantage in order to reduce undercuts, and to improve aesthetics and stability of the final product.

Tools 1024 for designing the coupling setting, insertion direction and the like are provided in the ninth and tenth user interface sessions 1021 and 1022.

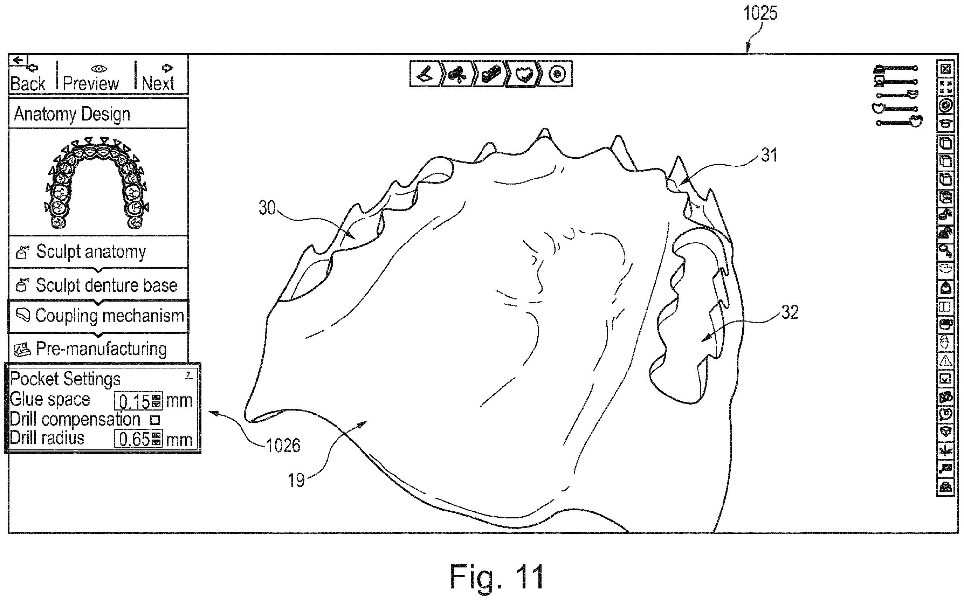

In the eleventh user interface session 1025 shown in Fig. 11 the intermediate denture teeth models 20, 21 and 22 are subtracted from the intermediate denture base model 19 using a Boolean subtraction. This creates first, second and third recesses 30, 31 and 32.

A pocket setting tool 1026 is provided in the user interface. This tool allows the user to determine a glue space which provides a small offset in the recesses to allow for gluing the manufactured denture base and teeth models together. Additionally, drill compensation can be selected, if the denture is to be manufactured by milling.

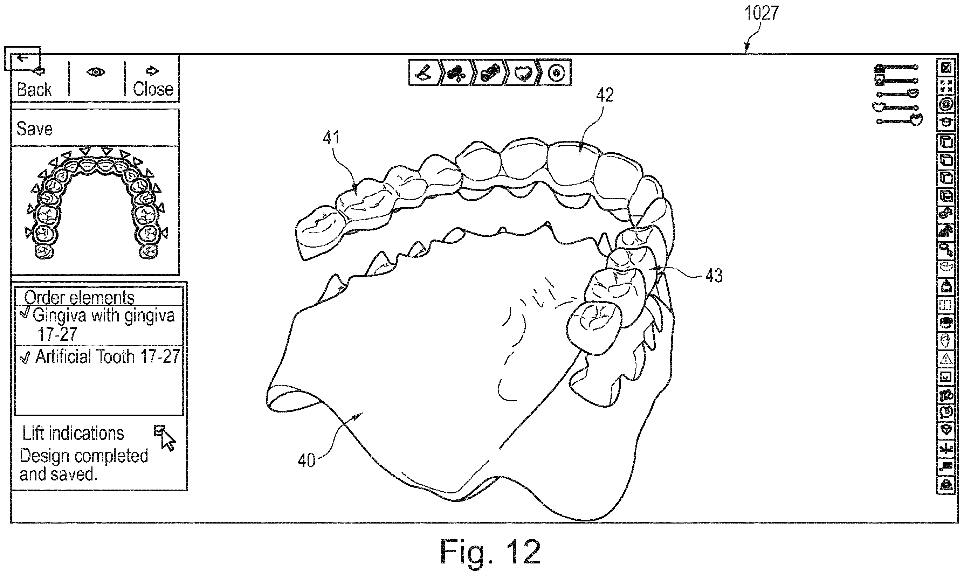

Finally, as shown in the twelfth user interface session 1027 in Fig. 12 all the models are finalized for production, providing a final denture base model 40, a first final denture teeth model 41, a second final denture teeth model 42 and third final denture teeth model 43.

These final models can then the shipped to a 3D manufacturing setup such as printing or milling manufacturing setups.

A claim may refer to any of the preceding claims, and "any" is understood to mean "any one or more" of the preceding claims.

The term "obtaining" as used in this specification may refer to physically acquiring for example medical images using a medical imaging device, but it may also refer for example to loading into a computer an image or a digital representation previously acquired.

It should be emphasized that the term "comprises/comprising" when used in this specification is taken to specify the presence of stated features, integers, steps or components but does not preclude the presence or addition of one or more other features, integers, steps, components or groups thereof.

The features of the method described above and in the following may be implemented in software and carried out on a data processing system or other processing means caused by the execution of computer-executable instructions. The instructions may be program code means loaded in a memory, such as a RAM, from a storage medium or from another computer via a computer network. Alternatively, the described features may be implemented by hardwired circuitry instead of software or in combination with software.

Although some embodiments have been described and shown in detail, the invention is not restricted to them, but may also be embodied in other ways within the scope of the subject matter defined in the following claims. In particular, it is to be understood that other embodiments may be utilized and structural and functional modifications may be made without departing from the scope of the present invention.

## Claims

1. A user interface presented on a digital monitor for grouping teeth models on a digital dental model, the user interface comprising a first user interface session including a rendering of the digital dental model in a first three dimensional workspace and a toolbar comprising at least one grouping button on the toolbar, wherein when activating the at least one grouping button at least one closed spline is generated enclosing at least one tooth at a boundary between at least one tooth model and a gingiva model of the digital dental model.

2. A user interface according to claim 1, wherein a plurality of grouping buttons are arranged in the toolbar and when activated each of the grouping button will provide one or more of the following actions:
- generate a plurality of closed splines, each spline enclosing one tooth model of the digital dental model,
- generate one closed spline enclosing all teeth models of the digital dental model,
- generate a plurality of closed splines, each spline enclosing a group of tooth models of the digital dental model, and/or
- prompting the user to select one or more tooth models of the digital dental model to be enclosed by a spline of the at least one closed spline.

3. A user interface according to any of the preceding claims, wherein the at least one tooth model is a denture teeth model and the digital dental model is a fitted existing denture model comprising the denture teeth model and a denture base model.

4. A user interface according to any of the preceding claims, further comprising a second user interface session comprising a rendering of the digital dental model in a second three dimensional workspace wherein the digital dental model is segmented into a denture base model and the at least one tooth model based on the at least one closed spline.

5. A user interface according to claim 4, wherein the digital dental model is segmented into the denture base model and the at least one tooth model based on the at least one closed spline, by using the at least one closed spline as a cutting spline to cut the digital dental model into the denture base model and the at least one tooth model.

6. A computer implemented method for generating a digital model of a denture comprising a digital model of a denture base and at least one digital model of denture teeth, wherein the method comprises, in sequence, the steps of:
- obtaining a digital model of a fitted existing denture,
- segmenting the digital model of the fitted existing denture into an intermediate denture base model and at least one intermediate denture teeth model,
- modifying the anatomy of the at least one intermediate denture base model,
- designing a coupling arrangement between the intermediate denture base model and the at least one intermediate denture teeth model, wherein the coupling arrangement comprises at least one recess formed in the intermediate denture base model and a protrusion extending from the at least one intermediate denture teeth model shaped to match the at least one recess, and
- generating a digital data file of a final denture base model based on the intermediate denture base model and generating a digital data file of at least one final denture teeth model based on the at least one intermediate denture teeth model.

7. A computer implemented method according to claim 6, wherein the step of obtaining a digital model of the fitted existing denture comprises scanning the fitted existing denture.

8. A computer implemented method according to any one or more of claims 6 or 7, wherein the step of segmenting comprises segmenting each tooth of the fitted existing denture in the digital model of the fitted existing denture.

9. A computer implemented method according to any one or more of claims 6-8, wherein the step of segmenting comprises segmenting a group of at least two teeth of the fitted existing denture in the digital model of the fitted existing denture.

10. A computer implemented method according to any one or more of claims 6-9, wherein the step of modifying the anatomy of the at least one intermediate denture base model comprises sculpting a virtual papilla.

11. A computer implemented method according to any one or more of claims 6-10, wherein the step of designing a coupling arrangement comprises providing a cement gap between the at least one recess and the corresponding protrusion in the shape of an offset between a digital model of the at least one recess and a digital model of the at least one protrusion.

12. A method for manufacturing a denture comprising the computer implemented method according to any one of the claims 6-11, further comprising providing the digital data file of the final denture base model and the digital data file of the at least one final denture teeth model to a 3D manufacturing process for manufacturing a denture base and at least one denture teeth and assembling the denture base and the at least one denture teeth by gluing.

13. A method for manufacturing a denture according to claim 12, wherein the 3D manufacturing process involves printing or milling.
